# EUROPÄISCHE PATENTSCHRIFT

(11) **EP 3 089 649 B1**
(45) Veröffentlichungstag und Bekanntmachung des Hinweises auf die Patenterteilung: **03.01.2018**
(21) Anmeldenummer: 15708159.7
(22) Anmeldetag: 26.02.2015
(51) Int. Cl.: A61B 1/018, A61B 1/04, A61B 1/06, A61B 5/107, G01B 11/22, G02B 23/24, G02B 27/09, G02B 27/42, A61B 1/00, A61B 5/00, A61B 1/07

(54) **ENDOSKOP MIT TIEFENBESTIMMUNG**
ENDOSCOPE HAVING DEPTH DETERMINATION
ENDOSCOPE À DÉTERMINATION DE PROFONDEUR

(30) Priorität: 07.03.2014 DE 102014204243
(43) Veröffentlichungstag der Anmeldung: 09.11.2016
(73) Patentinhaber: Siemens Aktiengesellschaft, 80333 München (DE)
(72) Erfinder: SCHICK, Anton, 84149 Velden (DE); RENTSCHLER, Peter, 75382 Neuhengstett (DE)
(86) Internationale Anmeldenummer: PCT/EP2015/054040
(87) Internationale Veröffentlichungsnummer: WO 2015/132127

(56) Entgegenhaltungen:
- WO-A2-2010/090837
- DE-A1- 10 308 383
- US-A1- 2007 188 855
- US-A1- 2010 045 778

## Beschreibung

Die Erfindung betrifft ein Endoskop zur Tiefenbestimmung eines Teilbereiches eines Hohlraumes.

Die Anzahl minimalinvasiver Operationen nimmt in den letzten Jahren stetig zu. Hierbei spielen Endoskope (3D-Endoskope), die eine Tiefenbestimmung eines in einem Patienten zu untersuchenden Hohlraumes und gleichzeitig ein bildgebendes Verfahren ermöglichen, eine immer größere Rolle. Nach dem Stand der Technik werden für die Tiefenbestimmung, beispielsweise eines Bauchraumes des Patienten, eine Mehrzahl von Zugängen (Ports) gelegt. Jedoch wird in der minimalinvasiven Chirurgie versucht, die Anzahl der Zugänge zu minimieren.

Um ein Minimum an Zugängen zu erreichen wird versucht, nach dem Stand der Technik bekannte Endoskope derart zu ergänzen, dass eine Tiefenbestimmung grundsätzlich ermöglicht wird. Ein wesentlicher Nachteil solcher 3D-Endoskope ist, dass nur wenig Bauraum für den Aufbau und die Integration einer Optik für die Tiefenbestimmung zur Verfügung steht. Dadurch werden insbesondere die Auflösung, die Bildqualität, die Tiefenschärfe und das Gesichtsfeld der Optik beeinträchtigt, wodurch sich insgesamt die optische Leistungsfähigkeit der Tiefenbestimmung bekannter 3D-Endoskope verringert. Die Schrift US 2010/045778 beschreibt ein solches Standard 3D-Endoskop gemäß der Präambel von Anspruch 1. Nach dem Stand der Technik bekannte 3D-Endoskope verwenden typischerweise das Prinzip der Stereoskopie zur Tiefenbestimmung. Hierzu werden zwei Abbildungskanäle, die jeweils eine Abbildungsoptik umfassen, durch das 3D-Endoskop geführt. Aus den Abbildern des Hohlraumes, die mittels der zwei Abbildungskanäle aus verschiedenen Blickwinkeln erfasst werden, kann die Tiefenbestimmung des Hohlraumes aus der Differenz von Bildpunkten der Abbilder gelingen. Ein grundsätzliches Problem der Stereoskopie ist das Korrespondenzproblem. Aus einem Bildpunkt des ersten Abbildes, das mittels des einen Abbildungskanals abgebildet wurde, und der veränderten Position des Bildpunktes auf einem zweiten Abbild, das mittels des anderen Abbildungskanals abgebildet wurde, ergibt sich die Tiefenbestimmung. Hierbei müssen der Bildpunkt im ersten Abbild und der Bildpunkt im zweiten Abbild als derselbe Bildpunkt erkennbar sein. Liegt diese Erkennbarkeit nicht vor, so besteht ein Korrespondenzproblem.

Bei gering texturierten Oberflächen, beispielsweise Blut oder bei organischem Gewebe, stehen typischerweise nur eine geringe Anzahl von Bildpunkten zur Verfügung, so dass das Korrespondenzproblem bei einer Verwendung der Stereoskopie in der minimalinvasiven Chirurgie verschärft wird.

Zur Lösung des Korrespondenzproblems schlägt der Stand der Technik sogenannte aktive Triangulationsverfahren vor, wobei bei einer aktiven Triangulation ein Abbildungskanal des 3D-Endoskops durch einen Projektionskanal ersetzt wird. Hierdurch wird zwar das Korrespondenzproblem größtenteils gelöst, aber nachteilig die optische Leistungsfähigkeit der Abbildungsoptik des 3D-Endoskops reduziert. Eine solche Reduktion ist besonders in der minimalinvasiven Chirurgie nicht zulässig.

Auch farbcodierte Triangulationsverfahren zur Tiefenbestimmung erweisen sich als problematisch, da die organischen Gewebe typischerweise von Blut umgeben sind, so dass eine annähernd vollständige Absorption von blauen und grünen Anteilen des projizierten Farbmusters erfolgt. Dadurch entstehen Fehlstellen im Abbild des Farbmusters, die wiederum zu einem Korrespondenzproblem führen.

Der vorliegenden Erfindung liegt folglich die Aufgabe zugrunde, die optische Tiefenbestimmung eines Endoskops zu verbessern.

Die Aufgabe wird durch eine Vorrichtung mit den Merkmalen des unabhängigen Anspruches 1 und durch ein Verfahren mit den Merkmalen des unabhängigen Anspruches 13 gelöst. In den abhängigen Ansprüchen sind vorteilhafte Ausgestaltungen und Weiterbildungen der Erfindung angegeben.

Das erfindungsgemäße Endoskop zur Tiefenbestimmung eines Teilbereiches eines Hohlraumes umfasst wenigstens einen Projektionskanal zur Projektion eines Musters auf eine Oberfläche des Hohlraumes und wenigstens einen Abbildungskanal, der zur optischen Abbildung eines von der Oberfläche des Hohlraumes reflektierten Abbildes des projizierten Musters vorgesehen ist, wobei der Projektionskanal wenigstens ein diffraktives optisches Element zur Erzeugung des Musters aufweist.

Erfindungsgemäß wird das Muster, welches die Tiefenbestimmung des Teilbereiches des Hohlraumes ermöglicht, mittels des diffraktiven optischen Elementes erzeugt. Diffraktive optische Elemente (abgekürzt DOE) sind optische Elemente, die zur räumlichen Strukturierung von Licht ausgebildet sind, wobei die Strukturierung mittels Beugung (Diffraktion) erfolgt. Beispielsweise ist ein optisches Gitter ein diffraktives optisches Element. Mittels des diffraktiven optischen Elements wird ein Muster, insbesondere ein Punktmuster, erzeugt, welches Muster nach einer Auswertung eine Tiefenbestimmung des Teilbereichs des Hohlraumes ermöglicht.

Durch die Anordnung des diffraktiven optischen Elements im Projektionskanal des Endoskops kann vorteilhafterweise ein DOE-Projektor, beispielsweise mittels weiterer optischen Komponenten, ausgebildet werden. Hierbei ist ein DOE-Projektor ein Projektor, der anstatt eines Dias ein diffraktives optisches Element umfasst. Besonders vorteilhaft ist, dass der benötigte Bauraumbedarf eines solchen DOE-Projektors - gegenüber Projektoren mit Dias - geringer ist.

Vorteilhafterweise wird durch den geringen Bauraumbedarf des DOE-Projektors, ein möglichst großer Abstand zwischen dem Projektionskanal und dem Abbildungskanal ermöglicht. Das ist deshalb von Vorteil, da der Abstand einer Triangulationsbasis der Triangulation entspricht, wobei die vergrößerte Triangulationsbasis insbesondere zu einer verbesserten Tiefenauflösung des Endoskops führt.

Erfindungsgemäß wird ein Verfahren zur Tiefenbestimmung eines Teilbereichs eines Hohlraumes vorgeschlagen, bei dem ein Endoskop mit einem Projektionskanal, der ein diffraktives optisches Element, einen Kollimator und eine Fokussierlinse, die zwischen dem Kollimator und dem diffraktiven optischen Element angeordnet ist, umfasst, und einem Abbildungskanal verwendet wird, wobei mittels des Projektionskanals ein Muster auf eine Oberfläche des Hohlraumes projiziert wird und ein von der Oberfläche reflektiertes Abbild des Musters mittels des Abbildungskanal abgebildet wird, wobei das Muster mittels des diffraktiven optischen Elements erzeugt wird.

Erfindungsgemäß wird ein mittels des diffraktiven optischen Elements erzeugtes Muster auf die Oberfläche des Teilbereiches des Hohlraumes projiziert und ein von der Oberfläche reflektiertes Abbild des Musters mittels des Abbildungskanals abgebildet. Es ergeben sich zum bereits genannten erfindungsgemäßen Endoskop gleichartige und gleichwertige Vorteile.

Erfindungsgemäß umfasst der Projektionskanal einen Kollimator und eine Fokussierlinse, wobei die Fokussierlinse zwischen dem Kollimator und dem diffraktiven optischen Element angeordnet ist.

Ein in den Projektionskanal eingeleitetes Licht wird mittels einer Linse kollimiert. Erfindungsgemäß umfasst der Projektionskanal eine weitere Linse, die das in den Projektionskanal eingeleitete Licht auf einen Arbeitsabstand des Endoskops fokussiert. Mit anderen Worten bildet die erstgenannte Linse einen Kollimator, eine Mehrzahl von Linsen eine Kollimatoroptik, und die zweitgenannte Linse die Fokussierlinse aus. Hierbei ist ein diffraktives optisches Element vorgesehen, das die Fokussierung des Lichtes bei der Erzeugung des Musters mit berücksichtigt.

Bevorzugt sind das diffraktive optische Element, der Kollimator und die Fokussierlinse in einem Teilbereich des Projektionskanals angeordnet, der bezüglich einer optischen Achse eine axiale Ausdehnung von höchstens 5 mm aufweist.

Hierbei verläuft die optische Achse im Teilbereich vorteilhafterweise koaxial mit einer Symmetrieachse des Projektionskanals. Es ist vorgesehen, dass das diffraktive optische Element, der Kollimator und die Fokussierlinse koaxial bezüglich der optischen Achse im Projektionskanal angeordnet sind. Durch die Anordnung des diffraktiven optischen Elements, des Kollimators und der Fokussierlinse im Teilbereich, der eine axiale Ausdehnung von höchstens 5 mm aufweist, wird ein im Projektionskanal des Endoskops angeordneter DOE-Projektor ausgebildet. Vorteilhafterweise weist dieser DOE-Projektor einen geringen Bauraumbedarf auf, so dass der DOE-Projektor in nach dem Stand der Technik bekannte Endoskope eingebaut werden kann. Bevorzugt ist hierzu ein Kollimator, der einen Durchmesser von höchstens 1 mm aufweist. Durch den genannten geringen Durchmesser des Kollimators kann vorteilhafterweise die Triangulationsbasis vergrößert werden, so dass die Tiefenauflösung des Endoskops verbessert wird.

Gemäß einer vorteilhaften Ausgestaltung der Erfindung ist eine Querschnittsfläche des Abbildungskanals größer als eine Querschnittsfläche des Projektionskanals.

Als Querschnittsfläche wird jeweils die Fläche bezeichnet, die sich durch einen Schnitt des Abbildungskanals oder des Projektionskanals senkrecht zur optischen Achse des jeweiligen Kanals ergibt.

Vorteilhafterweise genügt die gegenüber dem Abbildungskanal verringerte Querschnittsfläche des Projektionskanals zur Anordnung des DOE-Projektors im Projektionskanal. Durch den geringen Bauraumbedarf des DOE-Projektors wird Bauraum, der im Endoskop zur Verfügung steht, eingespart, so dass mehr Bauraum für den Abbildungskanal und folglich für eine Verbesserung der Abbildungsoptik, welche Abbildungsoptik im Abbildungskanal angeordnet ist, verwendet werden kann.

Bevorzugt ist eine Querschnittsfläche des Projektionskanals die kleiner gleich 2 mm² ist.

Dadurch wird vorteilhafterweise ein äußerst kleiner Projektionskanal ausgebildet, so dass folglich weiterer Bauraum im Endoskop eingespart werden kann. Hierbei weist der Abbildungskanal bevorzugt eine Querschnittsfläche von wenigstens 2 mm² auf. Insbesondere liegt die Querschnittsfläche des Abbildungskanals im Bereich von 25 mm² bis 64 mm², wobei größere Abbildungskanäle vorgesehen sein können.

Gemäß einer vorteilhaften Ausgestaltung der Erfindung ist der Projektionskanal optisch mit einer Monomodefaser gekoppelt.

Dadurch wird Licht, welches mittels der Monomodefaser (engl. Single-Mode Fiber; SMF) geführt wird, mittels der Monomodefaser in den Projektionskanal eingeleitet. Die Monomodefaser führt vorteilhafterweise nur eine Lichtmode, so dass Interferenzen zwischen mehreren Lichtmoden, die zu einer Störung des projizierten Musters führen könnten, vermieden werden.

Bevorzugt ist die Monomodefaser mit einem Laser gekoppelt, wobei das Licht des Lasers über die Monomodefaser in den Projektionskanal eingeleitet wird. Hierbei kann die Wellenlänge des Lasers zur optimalen Punktkontrasterzeugung, beispielsweise im blauen Spektralbereich, an die Anwendung in der Chirurgie angepasst sein. Besonders vorteilhaft ist, dass beispielsweise mit einem Interferenzfilter ein störender Einfluss von Tages- und/oder Kunstlicht durch die Verwendung eines Lasers als Lichtquelle verringert wird.

In einer bevorzugten Ausgestaltung der Erfindung ist der Abbildungskanal mit einer Kamera zur Aufnahme des Abbildes des reflektierten Musters optisch gekoppelt.

Besonders bevorzugt ist eine Kamera, die als Drei-Chip-Kamera ausgebildet ist. Hierbei weist die Kamera einen Chip für den roten, einen Chip für den grünen und einen Chip für den blauen Spektralbereich des aufgenommen Abbildes auf. Vorteilhafterweise wird dadurch ein annähernd vollständiges Abbild des reflektierten und mittels des Abbildungskanals abgebildeten Musters ermöglicht.

In einer bevorzugten Ausgestaltung der Erfindung umfasst das Endoskop wenigstens einen Instrumentierkanal.

Vorteilhafterweise können durch den Instrumentierkanal chirurgische Werkzeuge, die für die minimalinvasive Chirurgie benötigt werden, in den Hohlraum eingeführt werden. Durch die Anordnung eines diffraktiven optischen Elementes im Projektionskanal, wird Bauraum eingespart, der wiederum für den Instrumentierkanal verwendet werden kann.

Gemäß einer vorteilhaften Ausgestaltung der Erfindung wird mittels des diffraktiven optischen Elements ein Punktmuster erzeugt.

Hierbei entsprechen die einzelnen Punkte des Punktmusters den Beugungsordnungen des diffraktiven optischen Elements. Mit anderen Worten wird durch konstruktive und destruktive Interferenz des in den Projektionskanal eingeleiteten Lichtes ein Punktmuster mittels des diffraktiven optischen Elements erzeugt. Das Punktmuster wird auf die Oberfläche des Teilbereiches des Hohlraumes projiziert und ermöglicht durch eine Auswertung der Abstände der Punkte eine Tiefenbestimmung des Teilbereiches. Durch das Punktmuster, welches mittels des diffraktiven optischen Elements durch Beugung erzeugt wird, wird somit vorteilhafterweise das Korrespondenzproblem bei der aktiven Triangulation verringert.

Weitere Vorteile, Merkmale und Einzelheiten der Erfindung ergeben sich aus dem im Folgenden beschriebenen Ausführungsbeispielen sowie anhand der Zeichnungen. Dabei zeigen:
- Figur 1: einen Ausschnitt eines Projektionskanals eines Endoskops, wobei der Projektionskanal ein diffraktives optisches Element umfasst;
- Figur 2: eine Schnittdarstellung eines Endoskops mit einem Projektionskanal und einem Abbildungskanal; und
- Figur 3: eine weitere Schnittdarstellung eines Endoskops mit einem Projektionskanal und einem Abbildungskanal.

Gleichartige Elemente können in den Figuren mit denselben Bezugszeichen versehen sein.

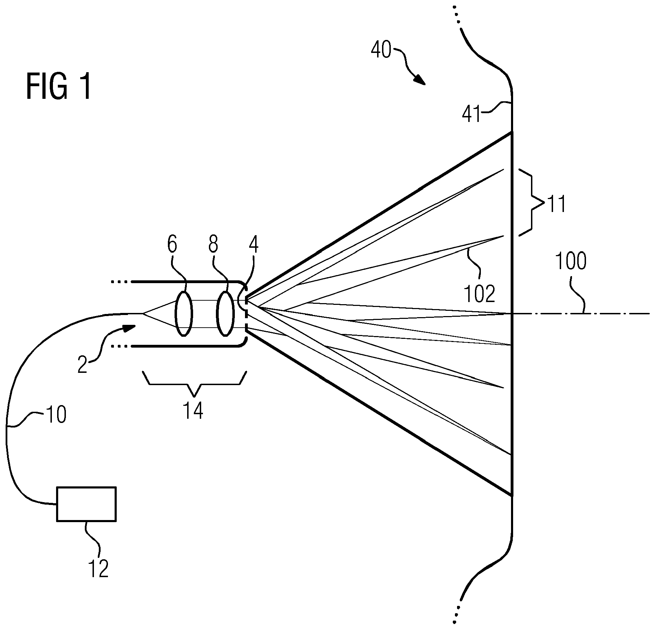

Figur 1 zeigt einen schematischen Ausschnitt eines Projektionskanals 2 eines nicht dargestellten Endoskops 1. Hierbei umfasst der Projektionskanal 2 erfindungsgemäß ein diffraktives optisches Element 4. Weiterhin sind ein Kollimator 6 und eine Fokussierlinse 8 innerhalb des Projektionskanals 2 angeordnet. Weitere optische Komponenten, beispielsweise Linsen, Spiegel, Objektive und/oder Strahlumlenkvorrichtungen können vorgesehen sein. Zudem kann eine Mehrzahl von Projektionskanälen vorgesehen sein. Es ist nicht zwingend, dass sich der Projektionskanal durch das gesamte Endoskop 1 erstreckt. Beispielsweise kann jeder Teilbereich des Endoskops 1, der wenigstens ein diffraktives optisches Element 2 umfasst, als Projektionskanal angesehen werden.

Der Kollimator 6, die Fokussierlinse 8 und das diffraktive optische Element 4 sind koaxial bezüglich einer optischen Achse 100 des Projektionskanals 2 angeordnet. Hierbei sind die genannten Elemente 4, 6, 8 in einem Teilbereich 14 des Projektionskanals 2 angeordnet, welcher Teilbereich 14 bezüglich der optischen Achse 100 eine axiale Ausdehnung von annähernd 3 mm aufweist. Durch die Ausbildung eines DOE-Projektors mittels des diffraktiven optischen Elements 4 im Projektionskanal 2 des Endoskops 1 kann Bauraum eingespart werden, der anderweitig, beispielsweise für einen nicht dargestellten Instrumentierkanal, verwendet werden kann.

Der Projektionskanal 2 ist mittels einer Monomodefaser 10 mit einem Laser 12 oder einer Leuchtdiode optisch gekoppelt. Das Licht des Lasers 12 wird in der Monomodefaser 10 geführt und in den Projektionskanal 2 eingeleitet, mittels des Kollimators 6 kollimiert und mittels der Fokussierlinse 8 fokussiert. Nach der Fokussierlinse 8 wird das Licht des Lasers 12 zum diffraktiven optischen Element 4 geleitet, so dass durch Beugung des Lichtes am diffraktiven optischen Element 4 ein Punktmuster auf einer Oberfläche 41 eines Teilbereiches eines Hohlraumes 40 projiziert wird. Die einzelnen Punkte des Punktmusters entsprechen hierbei den Beugungsordnungen 102 (Hauptmaxima und Nebenmaxima einer Intensitätsverteilung des gebeugten Lichtes).

Das diffraktive optische Element 4 ist derart ausgestaltet, dass die Abstände 11 der einzelnen Punkte des Punktmusters variieren, wobei mittels der Variation der Abstände 11 das Korrespondenzproblem gelöst beziehungsweise verringert wird. Mit anderen Worten gelingt eine Zuordnung der Punkte des reflektierten Musters über einen Vergleich eines ursprünglichen Punktmusters, welches ursprüngliche Punktmuster beispielsweise aus einer Projektion des Punktmusters auf eine ebene Oberfläche erzeugt wird (Kalibrierung). Die variierenden Abstände eines Punktes zu seinen benachbarten Punkten generieren folglich einen Code, der zur Auflösung beziehungsweise zur Verbesserung des Korrespondenzproblems herangezogen wird.

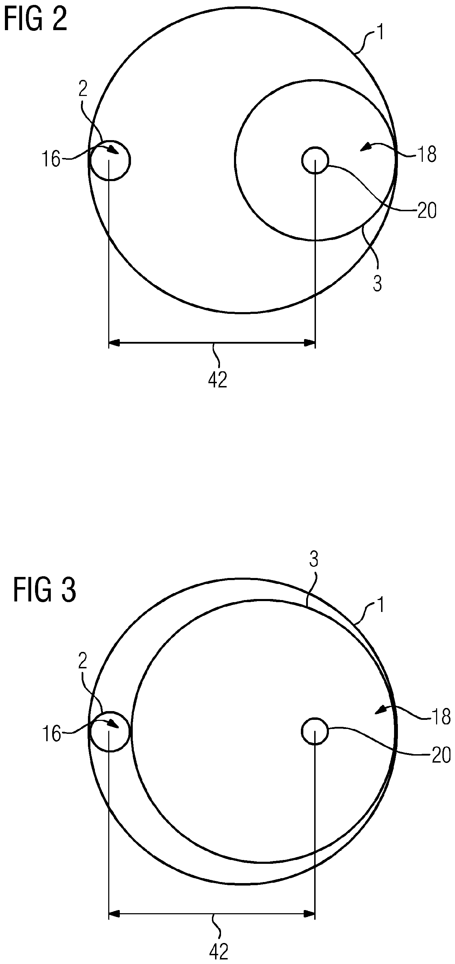

In Figur 2 ist eine schematische Schnittdarstellung eines Endoskops 1 gezeigt, wobei der Schnitt senkrecht zu einer optischen Achse 100 eines Projektionskanals 2 verläuft. Weiterhin zeigt Figur 2 einen Abbildungskanal 3, wobei eine Mehrzahl von Abbildungskanälen 2 vorgesehen sein kann. Bevorzugt ist ein Endoskop 1 mit zwei Abbildungskanälen 2 und einem Projektionskanal 2.

Durch die Anordnung oder Ausgestaltung eines DOE-Projektors im Projektionskanal 2 des Endoskops 1, kann eine Querschnittsfläche 16 des Projektionskanals 2 deutlich kleiner als eine Querschnittsfläche 18 des Abbildungskanals 3 sein. Durch die vergrößerte Querschnittsfläche 18 des Abbildungskanals 3 wird folglich die optische Leistungsfähigkeit einer nicht dargestellten Abbildungsoptik, die im Abbildungskanal 3 angeordnet ist, wesentlich verbessert.

Der Projektionskanal 2 ist möglichst an einem äußeren Randbereich des Endoskops 1 angeordnet. Weiterhin ist der Abbildungskanal 3 an einem weiteren äußeren Randbereich des Endoskops 1 angeordnet, der dem Projektionskanal 2 gegenüberliegt. Dadurch wird vorteilhafterweise eine Triangulationsbasis 42 zwischen einer Pupille 20 des Abbildungskanals 3 und dem Projektionskanal 2 vergrößert, wodurch die Tiefenauflösung des Endoskops 1 verbessert wird. Hierbei liegt die Triangulationsbasis in einem Bereich von 5 mm bis 10 mm.

In Figur 3 ist eine weitere schematische Schnittdarstellung eines Endoskops 1 dargestellt, wobei der Schnitt senkrecht zu einer optischen Achse 100 eines Projektionskanals 2 und/oder eines Abbildungskanals 3 verläuft. Das in Figur 3 schematisch illustrierte Endoskop 1 weist hierbei einen Durchmesser von wenigstens 10 mm auf. In Figur 3 ist die Querschnittsfläche 18 des Abbildungskanals 3 möglichst groß ausgestaltet, so dass der Abbildungskanal 3 annähernd vollständig den gesamten Bauraum des Endoskops 1 einnimmt. Das ist möglich, da der Projektionskanal 2 eine vergleichsweise geringe Querschnittsfläche 16 durch den DOE-Projektor beziehungsweise durch das diffraktive optische Element 4 benötigt, wobei der Vergleich zu Projektoren mit Dias zu ziehen ist. Dadurch wird insgesamt die optische Tiefenbestimmung und die optische Leistungsfähigkeit des Endoskops 1 weiter verbessert.

Der Projektionskanal 2 und/oder der Abbildungskanal 3 können weitere optische Komponenten, beispielsweise Linsen, Spiegel, Gitter, Strahlteiler und/oder Prismen und/oder gesamte optische Vorrichtungen, beispielsweise Objektive, umfassen. Insbesondere kann der Abbildungskanal 3 mittels eines Objektivs gebildet sein. Hierbei kann eine Kamera, beispielsweise eine Drei-Chip-Kamera am Objektiv angeordnet und/oder im Objektiv integriert sein. Eine Leitung der Abbilder erfolgt hierbei über optische Fasern, insbesondere mittels einer Monomodefaser 10.

Obwohl die Erfindung zum Teil durch die bevorzugten Ausführungsbeispiele näher illustriert und beschrieben wurden, so ist die Erfindung nicht durch die offenbarten Beispiele eingeschränkt oder andere Kombinationen können vom Fachmann hieraus abgeleitet werden, ohne den Schutzumfang der Erfindung zu verlassen.

## Patentansprüche

1. Endoskop (1) zur Tiefenbestimmung eines Teilbereiches eines Hohlraumes (40), umfassend wenigstens einen Projektionskanal (2) zur Projektion eines Musters auf eine Oberfläche (41) des Hohlraumes (40), und wenigstens einen Abbildungskanal (3), der zur Abbildung eines von der Oberfläche (41) des Hohlraumes (40) reflektierten Abbildes des projizierten Musters vorgesehen ist, wobei der Projektionskanal (2) wenigstens ein diffraktives optisches Element (4) zur Erzeugung des Musters, einen Kollimator (6) und eine Fokussierlinse (8) aufweist,
**dadurch gekennzeichnet, dass**
die Fokussierlinse (8) zwischen dem Kollimator (6) und dem diffraktiven optischen Element (4) angeordnet ist.

2. Endoskop (1) gemäß Anspruch 1, **dadurch gekennzeichnet, dass** das diffraktive optische Element (4), der Kollimator (6) und die Fokussierlinse (8) in einem Teilbereich (14) des Projektionskanals (2) angeordnet sind, wobei der Teilbereich (14) bezüglich einer optischen Achse (100) eine axiale Ausdehnung von höchstens 5 mm aufweist.

3. Endoskop (1) gemäß Anspruch 1 oder 2, **dadurch gekennzeichnet, dass** der Kollimator (6), die Fokussierlinse (8) und das diffraktive optische Element (4) koaxial bezüglich der optischen Achse (100) im Projektionskanal (2) angeordnet sind.

4. Endoskop (1) gemäß einem der vorangegangenen Ansprüche, **dadurch gekennzeichnet, dass** eine Querschnittsfläche (18) des Abbildungskanals (3) größer als eine Querschnittsfläche (16) des Projektionskanals (2) ist.

5. Endoskop (1) gemäß einem der vorangegangenen Ansprüche, **dadurch gekennzeichnet, dass** die Querschnittsfläche (16) des Projektionskanals (2) kleiner gleich 2 mm² ist.

6. Endoskop (1) gemäß einem der vorangegangenen Ansprüche, **dadurch gekennzeichnet, dass** die Querschnittsfläche (16) des Abbildungskanals (3) größer gleich 2 mm² ist.

7. Endoskop (1) gemäß einem der vorangegangenen Ansprüche, mit einem Projektionskanal (2), welcher optisch mit einer Monomodefaser (10) gekoppelt ist.

8. Endoskop (1) gemäß Anspruch 7, **dadurch gekennzeichnet, dass** die Monomodefaser (10) optisch mit einem Laser (12) gekoppelt ist.

9. Endoskop (1) gemäß einem der vorangegangenen Ansprüche, **dadurch gekennzeichnet, dass** der Abbildungskanal (3) mit einer Kamera zur Aufnahme des Abbildes des reflektierten Musters optisch gekoppelt ist.

10. Endoskop (1) gemäß Anspruch 9, **dadurch gekennzeichnet, dass** die Kamera eine Drei-Chip-Kamera ist.

11. Endoskop (1) gemäß einem der vorangegangenen Ansprüche, mit einem Instrumentierkanal.

12. Verfahren zur Tiefenbestimmung eines Teilbereiches eines Hohlraumes (40), bei dem ein Endoskop (1) mit einem Projektionskanal (2), der ein diffraktives optisches Element (4), einen Kollimator (6) und eine Fokussierlinse (8), die zwischen dem Kollimator (6) und dem diffraktiven optischen Element (4) angeordnet ist, umfasst, und einem Abbildungskanal (3) verwendet wird, wobei mittels des Projektionskanals (2) ein Muster auf eine Oberfläche (41) des Hohlrauems (40) projiziert wird und ein von der Oberfläche (41) reflektiertes Abbild des Musters mittels des Abbildungskanal (3) abgebildet wird, wobei das Muster mittels des diffraktiven optischen Elements (4) erzeugt wird.

13. Verfahren gemäß Anspruch 12, bei dem ein Punktmuster mittels des diffraktiven optischen Elementes (4) erzeugt wird.

14. Verfahren gemäß Anspruch 13, bei dem die Tiefenbestimmung des Teilbereiches des Hohlraumes (40) mittels der Abstände (11) von Punkten des Punktmusters erfolgt.

## Claims

1. Endoscope (1) for determining the depth of a portion of a cavity (40), comprising at least one projection channel (2) for projecting a pattern onto a surface (41) of the cavity (40) and at least one imaging channel (3) provided for imaging an image of the projected pattern reflected by the surface (41) of the cavity (40), wherein the projection channel (2) comprises at least one diffractive optical element (4) for generating the pattern, a collimator (6), and a focusing lens (8),
**characterized in that**
the focusing lens (8) is arranged between the collimator (6) and the diffractive optical element (4).

2. Endoscope (1) according to Claim 1, **characterized in that** the diffractive optical element (4), the collimator (6), and the focusing lens (8) are arranged in a portion (14) of the projection channel (2), wherein the portion (14) has an axial extent of at most 5 mm with respect to an optical axis (100).

3. Endoscope (1) according to Claim 1 or 2, **characterized in that** the collimator (6), the focusing lens (8), and the diffractive optical element (4) are arranged in the projection channel (2) in coaxial fashion with respect to the optical axis (100).

4. Endoscope (1) according to one of the preceding claims, **characterized in that** a cross-sectional area (18) of the imaging channel (3) is greater than a cross-sectional area (16) of the projection channel (2).

5. Endoscope (1) according to one of the preceding claims, **characterized in that** the cross-sectional area (16) of the projection channel (2) is less than or equal to 2 mm².

6. Endoscope (1) according to one of the preceding claims, **characterized in that** the cross-sectional area (16) of the imaging channel (3) is greater than or equal to 2 mm².

7. Endoscope (1) according to one of the preceding claims, comprising a projection channel (2) which is optically coupled to a single-mode fiber (10).

8. Endoscope (1) according to Claim 7, **characterized in that** the single-mode fiber (10) is optically coupled to a laser (10).

9. Endoscope (1) according to one of the preceding claims, **characterized in that** the imaging channel (3) is optically coupled to a camera for recording the image of the reflected pattern.

10. Endoscope (1) according to Claim 9, **characterized in that** the camera is a three-chip camera.

11. Endoscope (1) according to one of the preceding claims, comprising an instrumentation channel.

12. Method for determining the depth of a portion of a cavity (40), in which an endoscope (1) with a projection channel (2) comprising a diffractive optical element (4), a collimator (6), and a focusing lens (8) arranged between the collimator (6) and the diffractive optical element (4) and with an imaging channel (3) is used, wherein a pattern is projected onto a surface (41) of the cavity (40) by means of the projection channel (2) and an image of the pattern reflected by the surface (41) is imaged by means of the imaging channel (3), wherein the pattern is generated by means of the diffractive optical element (4).

13. Method according to Claim 12, in which a point pattern is generated by means of the diffractive optical element (4).

14. Method according to Claim 13, in which the depth determination of the portion of the cavity (40) is carried out by means of the distances (11) between points of the point pattern.

## Revendications

1. Endoscope (1) de détermination de la profondeur d'une région partielle d'une cavité (40), comprenant au moins un canal (2) de projection pour la projection d'un motif sur une surface (41) de la cavité (40), et au moins un canal (3) de reproduction, qui est prévu pour reproduire une image, réfléchie par la surface (41) de la cavité (40), du motif projeté, dans lequel le canal (2) de projection a au moins un élément (4) optique de diffraction pour produire le motif, un collimateur (6) et une lentille (8) de focalisation,
**caractérisé en ce que**
la lentille (8) de focalisation est montée entre le collimateur (6) et l'élément (4) optique de diffraction.

2. Endoscope (1) suivant la revendication 1, **caractérisé en ce que** l'élément (4) optique de diffraction, le collimateur (6) et la lentille (8) de focalisation sont disposés dans une région (14) partielle du canal (2) de projection, la région (14) partielle ayant, par rapport à un axe (100) optique, une étendue axiale de 5 mm au plus.

3. Endoscope (1) suivant la revendication 1 ou 2, **caractérisé en ce que** le collimateur (6), la lentille (8) de focalisation et l'élément (4) optique de diffraction sont disposés dans le canal (2) de projection coaxialement par rapport à l'axe (100) optique.

4. Endoscope (1) suivant l'une des revendications précédentes, **caractérisé en ce qu'**une surface (18) de section transversale du canal (8) de reproduction est plus grande qu'une surface (16) de section transversale du canal (2) de projection.

5. Endoscope (1) suivant l'une des revendications précédentes, **caractérisé en ce que** la surface (16) de section transversale du canal (2) de projection est inférieure ou égale à 2 mm².

6. Endoscope (1) suivant l'une des revendications précédentes, **caractérisé en ce que** la surface (16) de section transversale du canal (3) de reproduction est supérieure ou égale à 2 mm².

7. Endoscope (1) suivant l'une des revendications précédentes, comprenant un canal (2) de projection, qui est couplé optiquement à une fibre (10) monomode.

8. Endoscope (1) suivant la revendication 7, **caractérisé en ce que** la fibre (10) monomode est couplée optiquement à un laser (12).

9. Endoscope (1) suivant l'une des revendications précédentes, **caractérisé en ce que** le canal (3) de reproduction est couplé optiquement à un appareil photographique de prise de vue de l'image du motif réfléchi.

10. Endoscope (1) suivant la revendication 9, **caractérisé en ce que** l'appareil photographique est un appareil photographique à trois puces.

11. Endoscope (1) suivant l'une des revendications précédentes, comprenant un canal d'instrumentation.

12. Procédé de détermination de la profondeur d'une région partielle d'une cavité (40), dans lequel on utilise un endoscope (1) ayant un canal (2) de projection, qui renferme un élément (4) optique de diffraction, un collimateur (6) et une lentille (8) de focalisation, montée entre le collimateur (6) et l'élément (4) optique de diffraction, et un canal (3) de reproduction, dans lequel, au moyen du canal (2) de projection, on projette un motif sur une surface (41) de la cavité (40) et on reproduit une image, réfléchie par la surface (41), du motif au moyen du canal (3) de reproduction, le motif étant produit au moyen de l'élément (4) optique de diffraction.

13. Procédé suivant la revendication 12, dans lequel on produit un motif à points au moyen de l'élément (4) optique de diffraction.

14. Procédé suivant la revendication 13, dans lequel on effectue la détermination de profondeur de la région partielle de la cavité (40) au moyen des distances (11) entre des pointes du motif à points.
